(19) Europäisches Patentamt
European Patent Office
Office européen des brevets

(11) Veröffentlichungsnummer : **0 110 372**
**B1**

(12) **EUROPÄISCHE PATENTSCHRIFT**

(45) Veröffentlichungstag der Patentschrift : **10.09.86**

(21) Anmeldenummer : **83111922.7**

(22) Anmeldetag : **29.11.83**

(51) Int. Cl.⁴ : **C 07 D401/04**, C 07 D491/056, A 61 K 31/47// C07D213/38, C07D213/40, C07D213/50 ,(C07D491/056, 319:00, 221:00),(C07D491/056, 317:00, 221:00)

(54) **1-Phenylisochinolinderivate und Verfahren zu ihrer Herstellung, diese Verbindung enthaltende pharmazeutische Präparate und deren Anwendung.**

(30) Priorität : **02.12.82 DE 3244594**

(43) Veröffentlichungstag der Anmeldung : **13.06.84 Patentblatt 84/24**

(45) Bekanntmachung des Hinweises auf die Patenterteilung : **10.09.86 Patentblatt 86/37**

(84) Benannte Vertragsstaaten : **AT BE CH DE FR GB IT LI LU NL SE**

(56) Entgegenhaltungen :
**EP-A- 0 047 923**
**FR-A- 2 496 653**
**GB-A- 1 528 738**

(73) Patentinhaber : **HOECHST AKTIENGESELLSCHAFT**
**Postfach 80 03 20**
**D-6230 Frankfurt am Main 80 (DE)**

(72) Erfinder : **Konz, Elmar, Dr.**
**Brüningstrasse 9**
**D-6233 Kelkheim (Taunus) (DE)**
Erfinder : **Kruse, Hansjörg, Dr.**
**Feldbergstrasse 70**
**D-6233 Kelkheim (Taunus) (DE)**

**EP 0 110 372 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Substituierte 3-Hydroxymethylisochinoline haben nach DE-OS 22 46 307 spasmolytische Eigenschaften. Nach DE-OS 31 50 876 sind in 3-Stellung mit einer Alkylenaminogruppe substituierte 3,4-Dihydroisochinoline bekannt, die analgetische und antidepressive Wirkung besitzen. Isochinoline mit Pyridylsubstituenten in 3-Stellung (vgl. Eur. J. Med. Chem. *10*, 603 (1975)) sind gegen Mycoplasma gallisepticum aktiv. Dihydropyridine als Substituenten in 3-Stellung von Isochinolin werden in DE-OS 22 10 667 als Coronardilatatoren beschrieben.

Die vorliegende Erfindung betrifft neue in 3-Stellung mit einem basischen Ring substituierte 1-Phenylisochinolinderivate mit psychotropen Wirkungen, Verfahren zu ihrer Herstellung, diese Verbindungen enthaltende pharmazeutische Präparate und deren Anwendung.

Gegenstand der Erfindung sind 1-Phenylisochinolinderivate der allgemeinen Formel I

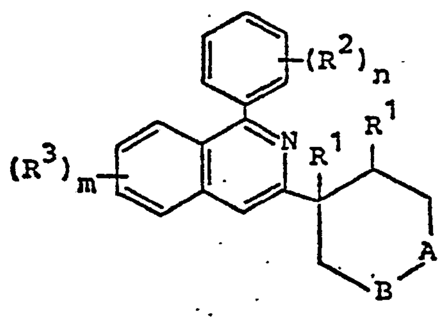

(I)

in der

m und n unabhängig voneinander eins oder zwei,

A und B eine $CH_2$- oder N—$R^4$-Gruppe, wobei $R^4$ Wasserstoff, Benzyl, ein geradkettiger verzweigter, $C_1$-$C_6$-Alkylrest oder ein geradkettiger oder verzweigter Alkenylrest mit bis zu 6 C-Atomen ist, bedeuten, und die Reste

$R^1$ Wasserstoff oder zusammen eine Bindung bedeuten, und

$R^2$ Wasserstoff, Halogen, Hydroxy, Nitro, Amino, $C_1$-$C_6$-Alkyl- oder $C_1$-$C_6$-Alkoxyreste bedeutet, und

$R^3$ Wasserstoff, Halogen, Hydroxy, Nitro, Amino, $C_1$-$C_6$-Alkyl- oder $C_1$-$C_6$-Alkoxyreste, Benzyloxy, Methylendioxy oder Äthylendioxygruppe bedeutet.

Bevorzugt sind solche Verbindungen der allgemeinen Formel I, in denen

m und n unabhängig voneinander eins oder zwei,

A und B eine $CH_2$- oder N—$R^4$-Gruppe, wobei $R^4$, Wasserstoff, ein geradkettiger oder verzweigter, $C_1$-$C_6$-Alkylrest oder ein geradkettiger oder verzweigter Alkenylrest mit bis zu 6 C-Atomen ist, bedeuten, und die Reste

$R^1$ Wasserstoff oder zusammen eine Bindung bedeuten, und

$R^2$ Wasserstoff, Halogen, Hydroxy, Nitro, Amino, einen $C_1$-$C_4$-Alkyl- oder $C_1$-$C_4$-Alkoxyreste und

$R^3$ Wasserstoff, Halogen, Hydroxy, Nitro, Amino, einen $C_1$-$C_4$-Alkyl- oder $C_1$-$C_4$-Alkoxyreste bedeuten.

Von besonderem Interesse sind Verbindungen der Formel I, in der

m und n eins oder zwei,

A und B eine $CH_2$- oder N—$R^4$-Gruppe, wobei $R^4$ Wasserstoff oder einen geradkettigen oder verzweigten $C_1$-$C_4$-Alkylrest, insbesondere der Methyl, Ethyl, Propyl, iso-Propyl, Butyl oder iso-Butylrest ist, die Reste

$R^1$ Wasserstoff oder zusammen eine Bindung bedeuten,

$R^2$ Wasserstoff, Fluor, Chlor, Brom, Hydroxy, Amino, Methyl, Ethyl, Methoxy oder Ethoxy, bevorzugt in ortho und/oder para-Position bedeutet und

$R^3$ Wasserstoff, Fluor, Chlor, Brom, Hydroxy, Amino, Methyl, Ethyl, Methoxy oder Ethoxy, bevorzugt in 6- und/oder 7-Position, bedeutet.

Von ganz besonderem Interesse sind Verbindungen der Formel I, in der

m und n eins,

A und B eine $CH_2$- oder N—$R^4$-Gruppe, wobei $R^4$, Wasserstoff, Methyl, Ethyl oder Propyl ist, die Reste

$R^1$ zusammen eine Bindung bedeuten,

$R^2$ Wasserstoff, Fluor, Chlor, Hydroxy, Methyl oder Methoxy bevorzugt in ortho-Position bedeutet und

$R^3$ Wasserstoff, Fluor, Chlor, Hydroxy, Methyl oder Methoxy bevorzugt in 6 und/oder 7-Position bedeutet.

Das Verfahren zur Herstellung der Verbindungen der allgemeinen Formel I ist dadurch gekennzeichnet, daß man

a) eine Verbindung der allgemeinen Formel II

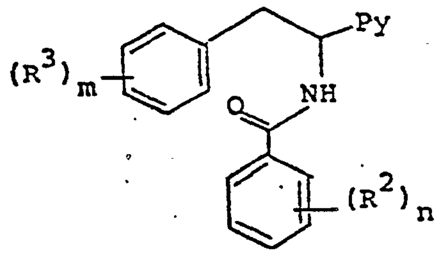

(II)

worin m, n, $R^2$ und $R^3$ die zur allgemeinen Formel I genannten Bedeutungen haben und Py den 3- oder 4-Pyridylrest bedeutet, mit einem wasserentziehenden Mittel wie z. B. Phosphorpentoxid oder Phosphoroxychlorid in einem hochsiedendem Lösungsmittel bei einer Temperatur zwischen 100 und 220 °C wie z. B. Tetralin, Diisopropylbenzol, Trimethylbenzol, Diphenyläther, Diethylenglykoldiethylether zu einem Isochinolinderivat der allgemeinen Formel III umsetzt.

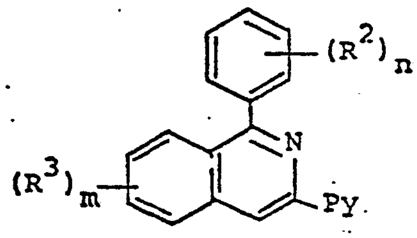

(III)

Anschließend wird mit einem Alkylierungsmittel der allgemeinen Formel Z—$R^4$ umgesetzt, worin Z Jod, Brom, Chlor, den Mesyl- oder Tosylrest bedeutet und $R^4$ Benzyl, einen geradkettigen oder verzweigten $C_1$-$C_6$-Alkylrest oder einen geradkettigen oder verzweigten Alkenylrest mit bis zu 6 C-Atomen bedeutet und die entstehenden quartären Pyridiniumsalze mit einem komplexen Metallhydrid reduziert zu Verbindungen der Formel I, worin die Reste $R_1$ zusammen eine Bindung darstellen und $R^4$ Benzyl, einen geradkettigen oder verzweigten, $C_1$-$C_6$-Alkylrest oder einen geradkettigen oder verzweigten Alkenylrest mit bis zu 6 C-Atomen bedeutet,
oder

b) eine Verbindung der allgemeinen Formel III katalytisch reduziert zu einer Verbindung der allgemeinen Formel I genannten Bedeutungen haben und die Reste $R^1$ und $R^4$ gleich Wasserstoff sind, und gegebenenfalls eine so erhaltene Verbindung der allgemeinen Formel I mit einem Alkylierungsmittel der allgemeinen Formel Z—$R^4$ umsetzt zu einer Verbindung der allgemeinen Formel I, worin m, n, $R^2$, $R^3$, A und B die zur Formel I genannten Bedeutungen haben und die Reste $R^1$ Wasserstoff und $R^4$ Benzyl, einen geradkettigen oder verzweigten $C_1$-$C_6$-Alkylrest oder einen geradkettigen oder verzweigten Alkenylrest mit bis zu 6 C-Atomen bedeuten.

Bei der Verfahrensweise a) werden die Verbindungen II entweder in einem Schritt bis zu vollaromatischen Isochinolinderivaten III umgesetzt, oder der Ringschluß erfolgt zuerst zur 3,4-Dihydroverbindung IV,

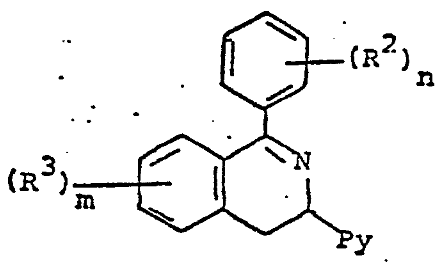

(IV)

worin m, n, $R^2$ und $R^3$ die zur Formel I genannte Bedeutung haben und Py den 3- oder 4-Pyridylrest bedeutet, und diese wird anschließend zum Isochinolinderivat III dehydriert, analog nach den Angaben von A. Pictet und F. W. Kay in Ber. *42*, 1973-1989 (1909) oder E. Späth, F. Berger und W. Kuntara, Ber. *63*, 134-141 (1930).

Die Verbindungen III werden dann mit dem Alkylierungsmittel Z—$R^4$ alkyliert. Die Reduktion der so entstehenden quartären Pyridiniumsalze wird vorteilhaft mit Lithiumaluminiumhydrid, Natriumcyanoborhydrid oder Natriumborhydrid in einem Lösungsmittel wie Ether, Tetrahydrofuran, Ethanol, Wasser oder in einem Gemisch dieser Lösungsmittel vorgenommen, bei Temperaturen zwischen 0° und 100 °C. Weiter ist es von Vorteil, Basen wie Natriumhydroxid zuzusetzen, wie in der Literatur beschrieben (Synthesis *1979*, 281 ; J. Am. Chem. Soc. *102*, 1064 (1980) ; Ann. *1978*, 1963).

Zur Herstellung derjenigen Verbindungen der Formel I, in denen m, n, $R^2$, $R^3$ die zur Formel I genannte Bedeutung haben, die Reste $R_1$ zusammen eine Bindung darstellen und $R^4$ ein Wasserstoffa-

tom bedeutet, wird eine Verbindung der allgemeinen Formel V mit Chlorameisensäurephenylester in das Urethan VI

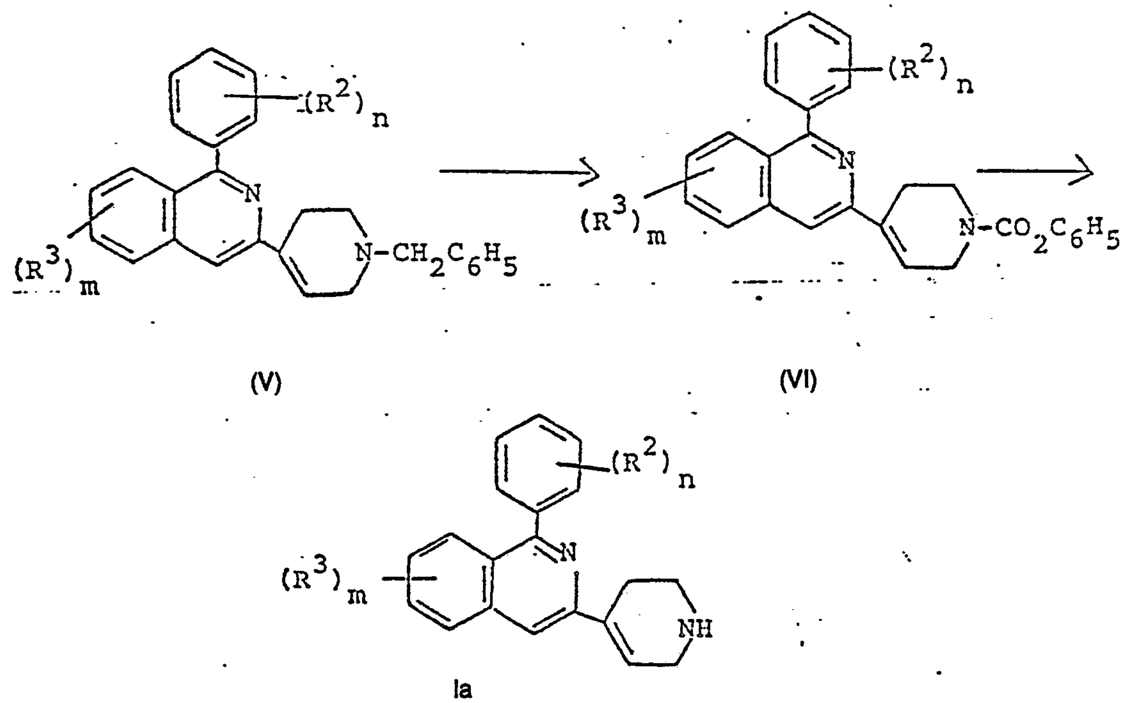

(V) (VI)

Ia

übergeführt und dieses anschließend mit verdünnter Natronlauge zu einer Verbindung der allgemeinen Formel Ia gespalten. Diese Reaktionsfolge kann mit den entsprechenden 1,2,5,6-Tetrahydro-pyrid-3-yl-Verbindungen ebenfalls durchgeführt werden und führt zu Verbindungen der allgemeinen Formel Ib, in denen die Reste $R_1$ zusammen eine Bindung darstellen und $R^4$ ein Wasserstoffatom bedeutet.

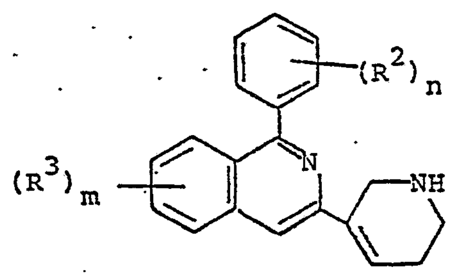

Ib

Diese Reaktion erfolgt analog J. Org. Chem. *26*, 4057, (1961) und J. Med. Chem. *21*, 309, (1978).

Bei der Verfahrensweise b) werden die Verbindungen der allgemeinen Formel III katalytisch hydriert, z. B. mit Palladium auf Tierkohle oder mit Platinoxid als Katalysator in äthanolischer Salzsäure bei Raumtemperatur und Normaldruck, bis zur Aufnahme der theoretischen Menge Wasserstoff. Dabei bilden sich als Nebenprodukte wechselnde Mengen der Tetrahydroisochinolinverbindung VII, worin n, m, $R^2$, $R^3$, A und B die zur Formel I genannte Bedeutung haben und wobei die Gruppe N—$R^4$ gleich N—H ist,

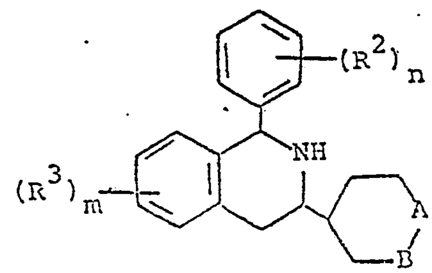

(VII)

die sich durch Säulenchromatographie z. B. an Silicagel mit Chloroform/Methanol abtrennen lassen.

Die Ausgangsmaterialien der allgemeinen Formel II werden wie folgt dargesellt aus 3-Cyanopyridin wird mit Benzylmagnesiumchlorid 3-Pyridylbenzylketon erhalten (vgl. J. Am. Chem. Soc. *78*, 674-676 (1956)).

Die analoge Reaktion mit 4-Cyanopyridin ergibt das 4-Pyridylbenzylketon.

Die gleichen Ketone können durch die in J. Med. Chem. *12*, (1969), 851-854 beschriebene Kondensation von Phenylessigsäureethylester bzw. von Benzylcyaniden mit Nikotinsäureethylester bzw. Isonikotinsäureethylester erhalten werden.

Die so erhaltenen Ketone reagieren mit Hydroxylamin zu den Oximen, die katalytisch reduziert werden zu den Aminen. Die Umsetzung mit den entsprechenden Säurechloriden ergibt die Amide der Formel II.

Die erfindungsgemäßen Verbindungen der allgemeinen Formel I zeigen Wirkungen auf das Zentralnervensystem. Sie heben insbesondere die durch Tetrabenazin verursachte Ptosis bei Mäusen auf. Des weiteren hemmen diese Verbindungen die Wiederaufnahme von Noradrenalin in Synaptosomen. Aufgrund dieser Eigenschaften können die erfindungsgemäßen Verbindungen als Wirkstoffe in antidepressiv wirkenden Arzneimitteln verwendet werden.

Zur Beurteilung der Wirkungen der Verbindungen der Erfindung wurden pharmakologische Versuche wie folgt durchgeführt :

A. Akute Toxizität der Maus nach intraperitonealer Applikation ($ALD_{50}$)

Als Versuchstiere wurden männliche Mäuse (Gassner, NMRI) mit einem Körpergewicht von 20-30 g verwendet. Je 6 Tiere wurden in einer Gruppe getestet. Die Verbindungen wurden in 1 %iger Methylhydroxy-ethyl-cellulose (MH) suspendiert und den Tieren intraperitoneal (i. p.) in einem Volumen von 10 ml/kg Körpergewicht verabreicht. Die Verbindungen gemäß der Erfindung wurden in Dosen von 3, 10, 30 und 100 mg/kg i. p. gegeben.

Die akute letale Dosis ($ALD_{50}$) wurde graphisch aus der Anzahl der innerhalb von 24 Stunden nach Applikation der Verbindung verstorbenen Tieren ermittelt. Sie ist in der folgenden Tabelle 1 aufgeführt.

B. Verhütung der durch Tetrabenazin induzierten Ptosis bei Mäusen nach intraperitonealer Applikation

Als Versuchstiere wurden männliche Mäuse (Gassner, NMRI) mit einem Körpergewicht von 20-25 g verwendet. Je 5 Tiere wurden in einer Gruppe getestet. Die Verbindungen wurden in 1 %iger Methylhydroxy-ethyl-cellulose suspendiert und den Tieren intraperitoneal (i. p.) in einem Volumen von 10 ml/kg Körpergewicht verabreicht. Die Verbindungen gemäß der Erfindung wurden in Dosen von 5, 10 und 20 mg/kg i. p. getestet. Die Kontrollgruppe erhielt lediglich 10 ml/kg MH i. p. ohne Wirkstoff.

Die Verbindungen wurden den Tieren 30 Minuten vor der Tetrabenazin-Gabe (TBZ) i. p. verabreicht. Die Kontrollgruppe erhielt lediglich 1 %iger MH und TBZ nach dem gleichen Zeitplan wie die mit den Testsubstanzen behandelten Tiere.

30 Minuten nach der TBZ-Injektion (TBZ : 40 mg/kg i. p.) wurden die Tiere einzeln in Plastikkästen gesetzt und 1 Minute später nach folgendem Schema die Ptosis festgestellt :

Ptosis Index :

| | | |
|---|---|---|
| Augen geschlossen | = 4 | (100 % Ptosis) |
| Augen 3/4 geschlossen | = 3 | ( 75 % Ptosis) |
| Augen 1/2 geschlossen | = 2 | ( 50 % Ptosis) |
| Augen 1/4 geschlossen | = 1 | ( 25 % Ptosis) |
| Augen offen | = 0 | ( 0 % Ptosis) |

Als $ED_{50}$ wird die Dosis definiert, die den durchschnittlichen Ptosis-Index (maximal 4) um 50 % reduziert.

Die Ergebnisse sind in der folgenden Tabelle 1 zusammengefaßt.

C. Wiederaufnahmehemmung von Noradrenalin in Synaptosomen

Synaptosomen aus Rattenhirn werden nach der Methode von Whittaker (Handbook of Neurochemistry *2*, 327-364, Editor A. Lajtha ; London and New York, 1969) isoliert und die Monoamin-Aufnahme nach der Methode von Schacht und Heptner gemessen (Biochemical Pharmac. *23*, 3413-3422). Die $^{14}C$-Noradrenalin-Aufnahme wurde in einem Krebs-Henseleit-Bikarbonatpuffer pH 7,4 der 11 Millimol Glucose enthielt, gemessen. 2,5 ml der Synaptosomen-Suspension wurden mit markiertem Noradrenalin bei 37 °C inkubiert in Gegenwart oder ohne Testsubstanz. Die Inkubationszeit betrug 4 Minuten. Die weitere Aufnahme wurde dann durch Abkühlen mit Eis gestoppt. Um nicht-spezifische Adsorptionen auszuschließen, wurden Kontrollproben bei 0 °C unter sonst gleichen Bedingungen inkubiert.

Die aufgenommenen Noradrenalinmengen wurden mit Hilfe der Membranfiltrationstechnik mit einem

Millipore Sampling Manifold mit Cellulosenitratfiltern von 25 mm Durchmesser und 0,6 Mikrometer Porengröße gemessen. Die Synaptosomen wurden unter vermindertem Druck gesammelt und die Radioaktivität in einem Packard-Tricarb-Scintillationszähler bestimmt. Die Menge an angesammelten Noradrenalin wurde angegeben als Prozent Radioaktivität, die zur Inkubationsmischung zugesetzt wurde.

Die $IC_{50}$-Werte (inhibition concentration) der nachstehenden Tabelle 1 geben die Konzentration der Testsubstanzen an, welche die Aufnahme von $^{14}C$-Noradrenalin zu 50 % hemmen.

Die in den Versuchen erhaltenen Werte für die Toxizität der Tetrabenazin-Ptosis und die Wiederaufnahmehemmung einiger Verbindungen gemäß der Erfindung sind in der nachstehenden Tabelle 1 zusammengefaßt.

Tabelle 1

| Verbindung Beispiel | $ADL_{50}$ mg/kg i.p. | Tetrabenazin-Ptosis $ED_{50}$ mg/kg i.p. | Noradrenalin-Aufnahmehemmung $IC_{50}$ u Mol/l |
|---|---|---|---|
| 2 | 100 | 1,4 | - |
| 3 | 210 | 0,13 | 0,0056 |
| 6 | 100 | 0,79 | 0,0330 |
| 8 | 85 | 0,54 | 0,0100 |
| 11 | 300 | 0,49 | - |
| 13 | 300 | 0,50 | 0,0062 |
| 19 | 40 | 3,30 | 0,050 |
| 20 | 75 | 0,33 | 0,013 |
| 21 | 75 | 0,80 | 0,017 |
| 23 | 55 | 3,0 | - |
| 25 | 28 | 6,59 | - |
| 27 | 27 | 0,53 | - |

Die erfindungsgemäßen Verbindungen der allgemeinen Formel I und ihre Salze mit pharmakologisch verträglichen Säuren sind als Antidepressiva innerhalb eines breiten Dosierungsbereiches wirksam. Die Höhe der verabreichten Dosis ist selbstverständlich abhängig von der Art der gewünschten Behandlung, von der Anwendungsweise und von dem Zustand, vom Typ und von der Größe des zu behandelnden Säugetiers. Bei oraler Verabreichung werden befriedigende Ergebnisse mit Dosen von 0,1-50 mg an aktiver Substanz pro kg Tierkörpergewicht erzielt, beim Menschen variiert die tägliche Dosis zwischen 10-400 mg an aktiver Substanz pro Mensch, vorzugsweise zwischen 20-200 mg, wobei Einzeldosen von 10-100 mg, vorzugsweise ein bis dreimal täglich gegeben werden können. Für intravenöse oder intramuskuläre Anwendung beträgt die Dosis 2-150 mg, vorzugsweise 5-100 mg täglich.

Die Verbindungen können allein oder mit pharmazeutisch üblichen Hilfsstofen und/oder Trägerstoffen gemischt angewandt werden. Für eine orale Anwendungsform werden die Verbindungen durch übliche Methoden in geeignete Darreichungsformen gebracht, wie Tabletten, Steckkapseln, wäßrige, alkoholische oder ölige Lösungen. Als inerte Träger können z. B. Magnesiumcarbonat, Milchzucker oder Maisstärke verwendet werden. Als ölige Trägerstoffe bzw. Lösungsmittel kommen beispielsweise besonders pflanzliche Öle in Betracht wie Olivenöl oder Sonnenblumenöl.

Die Salze der Verbindungen gemäß der Erfindung werden z. B. mit folgenden Säuren gebildet :

Chlor-, Brom, oder Jodwasserstoffsäure, Phosphorsäure, Schwefelsäure, Methylschwefelsäure, Amidosulfonsäure, Salpetersäure, Ameisensäure, Essigsäure, Propionsäure, Bernsteinsäure, Weinsäure, Milchsäure, Malonsäure, Fumarsäure, Oxalsäure, Zitronensäure, Äpfelsäure, Schleimsäure, Benzoesäure, Salicylsäure, Acetylaminoessigsäure, 4,4'-Methylen-bis-(3-hydroxy-2-naphtholsäure) (Embonsäure), Naphthalin-1,5-disulfonsäure, Ascorbinsäure, Phenylessigsäure, p-Amino-salicylsäure, Hydroxyethansulfonsäure, Benzolsulfonsäure oder synthetische Harze, die saure Gruppen enthalten, z. B. solche mit Ionenaustauschwirkung. Als Lösungsmittel solcher Salze kommen z. B. in Frage Wasser, physiologische Kochsalzlösungen oder Alkohol, wie z. B. Ethanol, Propandiol oder Glycerin, daneben auch Zuckerlösungen wie z. B. Glukose- oder Mannitlösungen, oder auch eine Mischung aus den genannten Lösungsmitteln. Solche Lösungen eignen sich auch zur intravenösen Applikation.
Verfahren a.)

Beispiel 1

6,7-Dimethoxy-3-(1-methyl-1,2,5,6-tetrahydropyrid-3-yl)-1-phenylisochinolin

4,45 g 6,7-Dimethoxy-1-phenyl-3-(pyrid-3-yl)-isochinolin in 400 ml Aceton gelöst werden mit 2,8 g Methyljodid versetzt und 2 Tage bei Raumtemperatur gerührt. Der Niederschlag wird abfiltriert und ergibt 3,5 g der Pyridiniumverbindung, die in 300 ml Methanol und 580 mg Natriumhydroxid in 50 ml Wasser gelöst wird un bei 0 °C werden 1,1 g Natriumborhydrid in 2 Portionen zugegeben. Das Gemisch steht über Nacht bei Raumtemperatur, das Methanol wird im Vakuum abdestilliert und der Rückstand zwischen Toluol und Wasser verteilt. Aus der trockenen Toluolphase werden 2,5 g rötliches Öl isoliert. Die Chromatographie an Silicagel mit Chloroform-Methanol (95 : 5)-Gemisch ergibt 2.1 g leicht gelbliches Harz, daß durch ethanolische Salzsäure in 1,75 g kristallines Hydrochlorid mit Schmp. 203-207 °C übergeht.

Das Ausgangsmaterial wird wie folgt dargestellt.

6,7-Dimethoxy-1-phenyl-3-(pyrid-3-yl)isochinolin

5,0 g 3,4-Dihydro-6,7-dimethoxy-1-phenyl-3(pyrid-3-yl) isochinolin werden in 150 ml Diethylenglykoldiethylether unter einer Stickstoffatmosphäre mit 2,5 g Palladium auf Tierkohle (10 %) auf 160 °C für 2,5 Stunden erhitzt. Der Katalysator wird abfiltriert, die Lösung einrotiert und der Rückstand mit Ether gewaschen. Es werden 4,0 g der Isochinolinverbindung mit Schmp. 176-177 °C isoliert.

3.4-Dihydro-6,7-dimethoxy-1-phenyl-3-(pyrid-3-yl)isochinolin

1,8 g N-[2-(3,4-Dimethoxyphenyl)-1-(3-pyridyl)ethyl]benzoesäureamid werden in 20 ml Phosphoroxichlorid 5 Stunden bei 60 °C und 1,5 Stunden bei 120 °C gerührt. Der gelbe Niederschlag wird abgesaugt, in 200 ml Wasser gelöst, die Lösung mit Kaliumcarbonat alkalisch gestellt und zweimal mit Methylenchlorid extrahiert. Es werden 1.6 g Dihydroisochinolinverbindung mit Schmp. 180-182 °C isoliert.

N-[2-(3.4-Dimethoxyphenyl)-1-(3-pyridyl)ethyl]benzoesäureamid

14,9 g 2-(3.4-Dimethoxyphenyl)-1-(3-pyridyl)ethylamin und 12.1 g Triethylamin in 200 ml Chloroform werden unter Eiskühlung und 9.75 g Benzoylchlorid in 10 ml Chloroform versetzt. Es wird 4 Stunden bei Raumtemperatur nachgerührt, im Vakuum das Chloroform abdestilliert und der Rückstand mit Toluol und Wasser gewaschen. 18,5 g des Amids mit Schmp. 156-158 °C werden erhalten.

2-(3.4-Dimethoxyphenyl)-1-(3-pyridyl)ethylamin

23,1 g 3,4-Dimethoxybenzyl-3-pyridyl-keton werden in 150 ml Pyridin mit 12,5 g Hydroxylamin-Hydrochlorid in das Oxim überführt. Es werden 16,8 g Oxim mit Schmp. 119-121 °C isoliert. Das Oxim wird in 400 ml Isopropanol und 200 ml methanolischen Ammoniak gelöst und bei Raumtemperatur mit Raney-Nickel hydriert. Nach Aufnahme der theoretischen Menge Wasserstoff wird der Katalysator abfiltriert und die Lösung einrotiert. Das ölige Amin wird ohne weitere Reinigung in das Amid überführt.

3.4-Dimethoxylbenzyl-3-pyridylketon

Zu einer siedenden Natriumethylatlösung, dargestellt aus 6 g Natrium und 100 ml Ethanol, wird das Gemisch von 45,4 g Nicotinsäureethylester und 35,4 g 3.4-Dimethoxybenzylcyanid getropft. Die Mischung wird 5 Stunden am Rückfluß gehalten und nach dem Abkühlen in 1 l Wasser eingegossen. Der überschüssige Nicotinsäureester wird mit Toluol entfernt und die wässrige Lösung mit Eisessig neutralisiert, wobei 50,7 g α-Cyano-3,4-dimethoxybenzyl-3-pyridylketon kristallin ausfallen (Schmp. 149-152 °C). Dieses Cyanoketon (31,0 g) wird in 6 n Salzsäure bei 80-90 °C hydrolisiert. Das Hydrochlorid des 3,4-Dimethoxybenzyl-3-pyridylketon kristallisiert aus Ethanol mit Schmp. 188-191 °C und die freie Base zeigt einen Schmp. von 60-64 °C. Die Verbindung wird ohne weiter Reinigung zum Oxim umgesetzt.

Beispiel 2

3-(1-Ethyl-1,2,5,6-tetrahydropyrid-4-yl)-1-(2-methylphenyl)isochinolin

4,5 g 1-(2-Methylphenyl)-3-(pyrid-4-yl)isochinolin werden mit 2,5 g Ethyljodid in 50 ml Ethanol in das

N-Ethylpyridiniumjodid überführt. Nach 7 Stunden Kochen wird das Ethanol abdestilliert und der Rückstand in 200 ml Methanol und 25 ml Wasser gelöst. Bei Raumtemperatur werden 1,2 g Natriumhydroxid in 25 ml Wasser und dann 1,7 g Natriumborhydrid in 2-Portionen zugegeben. Nach 6 Stunden bei Raumtemperatur wird wie in Beispiel 1 aufgearbeitet und chromatographiert. Die Base (2,8 g) wird als helles Öl isoliert und mit ethanolischer Salzsäure in das Hydrochlorid (1,6 g) mit Schmp. 176-179 °C überführt.

Das Ausgangsmaterial wird wie folgt dargestellt.

1-(2-Methylphenyl)-3-(pyrid-4-yl)isochinolin

37 g N-[2-Phenyl-1-(4-pyridyl)ethyl]-2-methylbenzoesäureamid in 800 ml Tetralin mit 240 g Phosphorpentoxid und 60 g Celite-Filterhilfsmittel zur besseren Verteilung 5 Stunden auf 180-200 °C erhitzt. Nach dem Abkühlen auf 120-140 °C werden weitere 120 g Phosphorpentoxid zugegeben und 16 Stunden auf 230 °C (Rückfluß) erhitzt. Nach dem Abkühlen wird das Tetralin abdekantiert und mit Toluol mehrmals gewaschen. Der gewaschene Rückstand wird in Toluol suspendiert und unter Rühren langsam mit Wasser versetzt. Die wässrige Phase wird abgetrennt und mit Kaliumhydroxid stark alkalisch gemacht. Das Produkt wird mit Toluol extrahiert und nach dem Abdestillieren des Toluols an Silicagel mit Chloroform Essigsäureethylester (8 : 2) chromatographiert. Es werden 25 g ölige Base isoliert, die mit ethanolischer Salzsäure ein Hydrochlorid mit Schmp. 255-259 °C vergibt.

N-[2-Phenyl-1-(4-pyridyl)ethyl]-2-methylbenzoesäureamid

56,0 g 2-Phenyl-1-(4-pyridyl)ethylamin und 60 g Triethylamin werden in 800 ml Chloroform unter Eiskühlung mit 48,0 g 2-Methylbenzoylchlorid versetzt. Die Mischung wird 2 Stunden bei Raumtemperatur nachgerührt und dann mit Natriumhydrogencarbonatlösung und Wasser gewaschen. Nach dem Trocknen der Chloroformlösung wird das Lösungsmittel abdestilliert und der ölige Rückstand mit Äther kristallisiert. Es werden 48 g Amid mit Schmp. 168-170 °C isoliert.

2-Phenyl-1-(4-pyridyl)ethylamin

5,34 g Benzyl-4-pyridyl-keton und 3,75 g Hydroxylaminhydrochlorid werden in 50 ml Pyridin 4 Stunden zum Sieden erhitzt. Nach dem Abkühlen wird in 600 ml Wasser eingerührt und das Oxim (5,6 g) mit Schmp. 194-196 °C abfiltriert. Das Oxim wird in Isopropanol mit Raney-Nickel bei 50 °C und Normaldruck mit Wasserstoff hydriert. Nach der üblichen Aufarbeitung erhält man das Amin als hellgelbes Öl.

Benzyl-4-pyridyl-keton

Aus 6.6 g Magnesiumspäne und 34,2 g Benzylchlorid wird nach bekannten Methoden die Benzylmagnesiumchlorid-Verbindung in Ether hergestellt. Unter Eiskühlung werden 26 g 4-Cyanopyridin in 200 ml Ether zur Grignard-Verbindung getropft, wobei ein dicker Kristallbrei entsteht, der 20 Stunden bei Raumtemperatur nachgerührt wird.

Die Reaktionsmischung wird langsam mit 50 ml Wasser und 100 ml 5 n Salzsäure hydrolysiert. Die wässrige Phase 1,5 Stunden auf dem Dampfbad erhitzt, mit Kaliumcarbonat alkalisch gestellt und mit Toluol extrahiert. Aus der Toluolphase wird nach Kristallisation aus Äther 5.6 g des Ketons mit Schmp. 94-96 °C isoliert.

Beispiel 3

1-(2-Methylphenyl)-3-(1,2,5,6-tetrahydropyrid-4-yl)isochinolin

Zu 7.7 g 3-(1-Benzyl-1,2,5-6-tetrahydropyrid-4-yl)-1-(2-methylphenyl)isochinolin und 8.1 g Triethylamin in 150 ml Chloroform werden bei 0 °C 12,5 g Chlorameisensäurephenylester getropft. Die Reaktionsmischung wird 15 Stunden bei Raumtemperatur gerührt. Nach dem Abdestillieren des Lösungsmittels wird der Rückstand zwischen Toluol und 0,2 n Natronlauge verteilt. Die Toluolphase wird eingeengt und der Rückstand in 200 ml Äthanol mit 200 ml 10 % Natronlauge 17 Stunden bei 60 °C hydrolysiert. Das Lösungsmittel wird wieder abdestilliert und der Rückstand in Toluol mit Wasser gewaschen. Man isoliert 4,5 g der amorphen Base, die mit ethanolischer Salzsäure 1,9 g des Hydrochlorids mit Schmp. 276-278 °C ergibt.

Analog zu den voranbeschriebenen Beispielen lassen sich die Verbindungen der Tabelle 2 und 3 darstellen.

(Siehe Tabelle 2 Seite 9 f.)

Tabelle 2

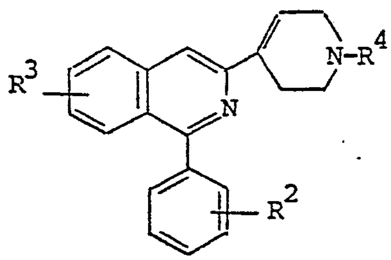

| Beispiel | $R^4$ | $R^2$ | $R^3$ | Salz | (Schmp. °C) |
|---|---|---|---|---|---|
| 4 | $CH_3$ | $2-CH_3$ | H | Hydrochlorid | 290 - 294 |
| 5 | $CH_3$ | H | H | Hydrochlorid | 195 - 198 |
| 6 | $CH_3$ | 2-Cl | H | Hydrochlorid | 191 - 193 |
| 7 | $CH_2-C_6H_5$ | $2-CH_3$ | H | Hydrochlorid | 279 - 281 |
| 8 | $CH_3$ | 2-F | H | Hydrochlorid | 208 - 211 |
| 9 | $C_4H_9$ | $2-CH_3$ | H | Hydrochlorid | 189 - 193 |
| 10 | $CH_3$ | H | 6-Cl | Hydrochlorid | 320 - 323 |
| 11 | $CH_2-CH=CH_2$ | $2-CH_3$ | H | Oxalat | 213 - 215 |
| 12 | $CH_2-CH=CH_2$ | H | H | Hydrochlorid | 260 - 262 |
| 13 | H | H | H | Hydrochlorid | 327 |
| 14 | $CH_3$ | H | $6,7-di-CH_3O$ | Hydrochlorid | 245 - 247 |
| 15 | H | H | $6,7-di-CH_3O$ | Hydrochlorid | 278 - 281 |

Tabelle 3

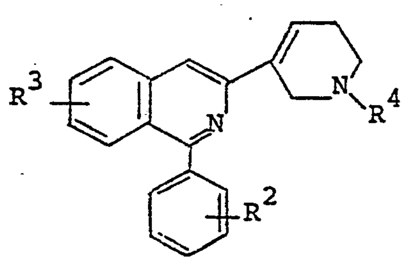

| Beispiel | $R^4$ | $R^2$ | $R^3$ | Salz | (Schmp. °C) |
|---|---|---|---|---|---|
| 16 | $CH_3$ | H | $6,7-di-CH_3O$ | Hydrochlorid | 203 - 207 |

(Fortsetzung)

| Beispiel | $R^4$ | $R^2$ | $R^3$ | Salz | (Schmp. °C) |
|---|---|---|---|---|---|
| 17 | $CH_2$-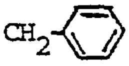 | H | 6,7-di-$CH_3O$ | Oxalat | 239 - 240 |
| 18 | H | H | 6,7-di-$CH_3O$ | Hydrochlorid | 224 - 227 |
| 19 | $CH_3$ | H | H | Hydrochlorid | 219 - 221 |
| 20 | H | H | H | Hydrochlorid | 239 - 240 |

Verfahren b.)

Beispiel 21

1-Phenyl-3-(piperidin-4-yl)-isochinolin

7,7 g 1-Phenyl-3-(pyrid-4-yl)-isochinolin werden mit 0,5 g Platinoxid in 800 ml Ethanol bei Raumtemperatur und Normaldruck 24 Stunden hydriert. Der Katalysator wird abfiltriert, die Lösung einrotiert und der Rückstand zwischen Chloroform und Wasser verteilt. Die Chloroformlösung wird eingedampft und der Rückstand in Äther gelöst. Es kristallisieren 1,95 g 1-Phenyl-3-(piperidin-4-yl)-1,2,3,4-tetrahydroisochinolin mit Schmp. 141-142° aus.

Die Mutterlauge enthält 5,6 g eines Substanzgemisches, das durch Chromatographie getrennt wird. Man isoliert 2,8 g 1-Phenyl-3-(piperidin-4-yl)-isochinolin, das als Hydrochlorid bei 238-242 °C schmilzt.

Beispiel 22

1-(2-Methylphenyl)-3-(piperidin-4-yl)-isochinolin-Hydrochlorid mit Schmelzpunkt 235-236 °C wird in analoger Verfahrensweise wie in Beispiel 21 beschrieben aus 1-(2-Methylphenyl)-3-(pyrid.-4-yl)-isochinolin erhalten.

Beispiel 23

3-(1-Allylpiperidin-4-yl)-1-(2-methylphenyl)-isochinolin

Aus 1,3 g 1-(2-Methylphenyl)-3-(piperidin-4-yl)-isochinolin Hydrochlorid wird mit Kaliumcarbonat die Base freigesetzt und in 40 ml Toluol gelöst. Diese Lösung wird mit 0,82 g Natriumcarbonat, 0,1 g Kaliumjodid und 0,61 g Allylbromid 15 Stunden bei Raumtemperatur und dann noch 2 Stunden bei 50 °C gerührt. Die Reaktionsmischung wird zwischen Toluol und Wasser verteilt und aus der Toluollösung werden 1,2 g eines hellen Öls isoliert, das in Isopropanol mit Oxalsäure in 1,4 g des Oxalats mit Schmelzpunkt 217-219 °C überführt werden kann.

Analog zu den voran beschriebenen Beispielen 21-23 werden die Verbindungen der Tabelle 4 dargestellt

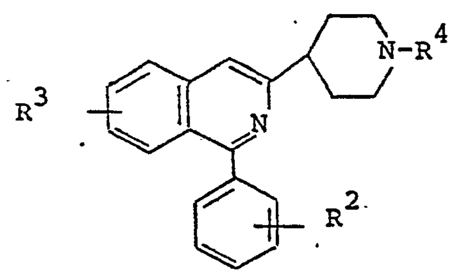

(Siehe Tabelle Seite 11 f.)

| Beispiel | $R^4$ | $R^2$ | $R^3$ | Salz (Schmp. °C) |
|---|---|---|---|---|
| 24 | $-C_2H_5$ | H | H | Hydrochlorid amorph |
| 25 | $-CH_3$ | H | H | Hydrochlorid 249 - 252 |
| 26 | $-C_2H_5$ | $2-CH_3$ | H | Hydrochlorid amorph |
| 27 | $-CH_3$ | $2-CH_3$ | H | Hydrochlorid 201 - 202 |

**Patentansprüche** (für die Vertragsstaaten : BE, CH, DE, FR, GB, IT, LI, LU, NL, SE)

1. 1-Phenylisochinolinderivate der allgemeinen Formel I

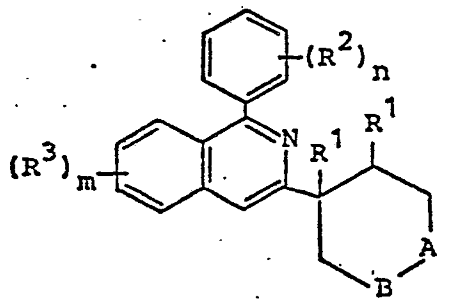

(I)

in der

m und n unabhängig voneinander eins oder zwei,

A und B eine $CH_2$- oder N—$R^4$-Gruppe, wobei $R^4$ Wasserstoff, Benzyl, ein geradkettiger oder verzweigter $C_1$-$C_6$-Alkylrest oder ein geradkettiger oder verzweigter Alkenylrest mit bis zu 6 C-Atomen ist, bedeuten, und die Reste

$R^1$ Wasserstoff oder zusammen eine Bindung bedeuten, und

$R^2$ Wasserstoff, Halogen, Hydroxy, Nitro, Amino, $C_1$-$C_6$-Alkyl- oder $C_1$-$C_6$-Alkoxyreste bedeutet, und

$R^3$ Wasserstoff, Halogen, Hydroxy, Nitro, Amino, $C_1$-$C_6$-Alkyl- oder $C_1$-$C_6$-Alkoxyreste, Benzyloxy, Methylendioxy oder Äthylendioxygruppe bedeutet.

2. Verfahren zur Herstellung einer Verbindung der allgemeinen Formel I in Anspruch 1, dadurch gekennzeichnet, daß man

a) eine Verbindung der allgemeinen Formel II

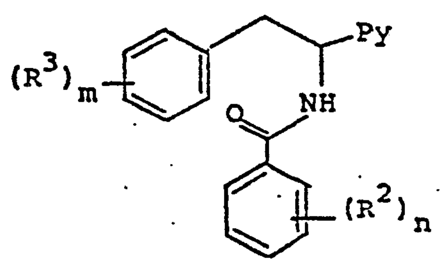

(II)

worin m, n, $R^2$ und $R^3$ die zur allgemeinen Formel I genannten Bedeutungen haben und Py den 3- oder 4-Pyridylrest bedeutet, mit einem wasserentziehenden Mittel in einem hochsiedenden Lösungsmittel bei einer Temperatur zwischen 100 und 220 °C zu einem Isochinolinderivat der allgemeinen Formel III umsetzt,

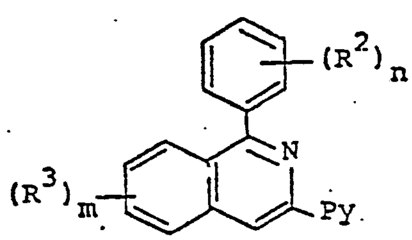

(III)

anschließend die so erhaltene Verbindung der allgemeinen Formel III mit einem Alkylierungsmittel Z—$R^4$ umsetzt, worin Z Jod, Brom, Chlor, den Mesyl- oder Tosylrest und $R^4$ Benzyl, einen geradkettigen oder verzweigten, $C_1$-$C_6$-Alkylrest oder einen geradkettigen oder verzweigten $C_2$-$C_6$-Alkenylrest mit bis zu 6 C-Atomen bedeuten, und das so erhaltene quarternäre Pyridiniumsalz mit einem komplexen Metallhydrid reduziert zu einer Verbindung der allgemeinen Formel I, worin die reste $R^1$ zusammen eine Bindung darstellen und $R^4$ Benzyl, einen geradkettigen oder verzweigten $C_1$-$C_6$-Alkylrest oder einen geradkettigen oder verzweigten $C_2$-$C_6$-Alkenylrest bedeutet,
oder

b) eine Verbindung der allgemeinen Formel III katalytisch reduziert zu einer Verbindung der Formel I, worin m, n, $R^2$, $R^3$, A und B die zur Formel I genannten Bedeutungen haben und die Reste $R^1$ und $R^4$ gleich Wasserstoff sind,
und gegebenenfalls eine so erhaltene Verbindung der allgemeinen Formel I mit einem Alkylierungsmittel der allgemeinen Formel Z—$R^4$ umsetzt, zu einer Verbindung der allgemeinen Formel I, worin m, n, $R^2$, $R^3$, A und B die zur Formel I genannten Bedeutungen haben und die Reste $R^1$ Wasserstoff und $R^4$ Benzyl, einen geradkettigen oder verzweigten $C_1$-$C_6$-Alkylrest oder einen geradkettigen oder verzweigten Alkenylrest mit bis zu 6 C-Atomen bedeuten.

3. Arzneimittel, gekennzeichnet durch einen Gehalt an einem 1-Phenylisochinolinderivat der allgemeinen Formel I in Anspruch 1.

4. 1-Phenylisochinolinderivate der allgemeinen Formel I in Anspruch 1 zur Verwendung bei Behandlung von Depressionen.

**Patentanspruch** (für den Vertragsstaat AT)

Verfahren zur Herstellung von 1-Phenylisochinolinderivaten der allgemeinen Formel I

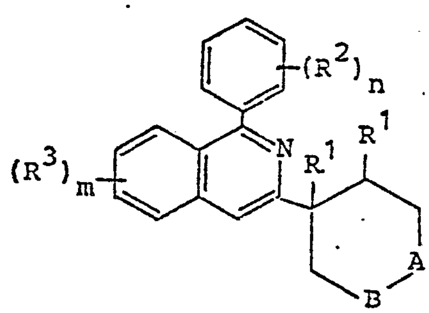

(I)

in der
m und n unabhängig voneinander eins oder zwei,
A und B eine $CH_2$- oder N—$R^4$-Gruppe, wobei $R^4$ Wasserstoff, Benzyl, ein geradkettiger oder verzweigter $C_1$-$C_6$-Alkylrest oder ein geradkettiger oder verzweigter Alkenylrest mit bis zu 6 C-Atomen ist, bedeuten und die Reste
$R^1$ Wasserstoff oder zusammen eine Bindung bedeuten, und
$R^2$ Wasserstoff, Halogen, Hydroxy, Nitro, Amino, $C_1$-$C_6$-Alkyl- oder $C_1$-$C_6$-Alkoxyreste bedeutet, und
$R^3$ Wasserstoff, Halogen, Hydroxy, Nitro, Amino, $C_1$-$C_6$-Alkyl- oder $C_1$-$C_6$-Alkoxyreste, Benzyloxy, Methylendioxy oder Äthylendioxygruppe bedeutet, dadurch gekennzeichnet, daß man

a) eine Verbindung der allgemeinen Formel II

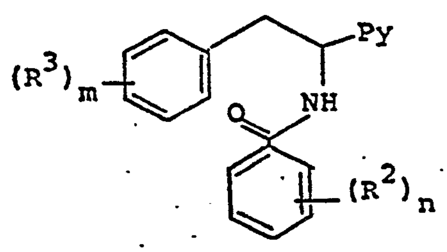

(II)

worin m, n, $R^2$ und $R^3$ die zur allgemeinen Formel I genannten Bedeutungen haben und Py den 3- oder 4-Pyridylrest bedeutet, mit einem wasserentziehenden Mittel in einem hochsiedenden Lösungsmittel bei einer Temperatur zwischen 100 und 220 °C zu einem Isochinolinderivat der allgemeinen Formel III umsetzt,

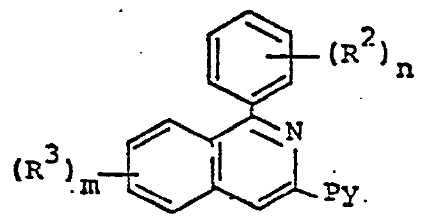

(III)

anschließend die so erhaltene Verbindung der allgemeinen Formel III mit einem Alkylierungsmittel Z—$R^4$ umsetzt, worin Z Jod, Brom, Chlor, den Mesyl- oder Tosylrest und $R^4$ Benzyl, einen geradkettigen oder verzweigten $C_1$-$C_6$-Alkylrest oder einen geradkettigen oder verzweigten $C_2$-$C_6$-Alkenylrest bedeuten, und das so erhaltene quarternäre Pyridiniumsalz mit einem komplexen Metallhydrid reduziert zu einer Verbindung der allgemeinen Formel I, worin die Reste $R^1$ zusammen eine Bindung darstellen und $R^4$ Benzyl, einen geradkettigen oder verzweigten $C_1$-$C_6$-Alkylrest oder einen geradkettigen oder verzweigten Alkenylrest mit bis zu 6 C-Atomen bedeutet,
oder

b) eine Verbindung der allgemeinen Formel III katalytisch reduziert zu einer Verbindung der Formel I, worin m, n, $R^2$, $R^3$, A und B die zur Formel I genannten Bedeutungen haben und die Reste $R^1$ und $R^4$ gleich Wasserstoff sind, und gegebenenfalls eine so erhaltene Verbindung der allgemeinen Formel I mit einem Alkylierungsmittel der allgemeinen Formel Z—$R^4$ umsetzt, zu einer Verbindung der allgemeinen Formel I, worin m, n, $R^2$, $R^3$, A und B die zur Formel I genannten Bedeutungen haben und die Reste $R^1$ Wasserstoff und $R^4$ Benzyl, einen geradkettigen oder verzweigten $C_1$-$C_6$-Alkylrest oder einen geradkettigen oder verzweigten Alkenylrest mit bis zu 6 C-Atomen bedeuten.

**Claims** (for the Contracting states : BE, CH, DE, FR, GB, IT, LI, LU, NL, SE)

1. A 1-phenylisoquinoline derivative of the formula I

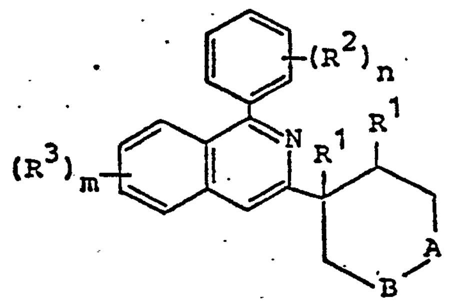

(I)

in which

m and n, independently of one another, denote one or two,

A and B denote a $CH_2$- or N—$R^4$ group, $R^4$ being hydrogen, benzyl or a straight-chain or branched $C_1$-$C_6$-alkyl radical, or a straight-chain or branched alkenyl-radical with up to 6 carbon atoms, and the radicals

$R^1$ denote hydrogen, or, together, a bond, and

$R^2$ denotes hydrogen, halogen, hydroxyl, nitro, amino, $C_1$-$C_6$-Alkyl or $C_1$-$C_6$-alkoxy radicals, and

$R^3$ denotes hydrogen, halogen, hydroxyl, nitro, amino, $C_1$-$C_6$-alkyl or $C_1$-$C_6$-alkoxy radical, or the benzyloxy, methylenedioxy or ethylenedioxy group.

2. A process for the preparation of a compound of the formula I in claim 1, which comprises

a) reacting a compound of the formula II

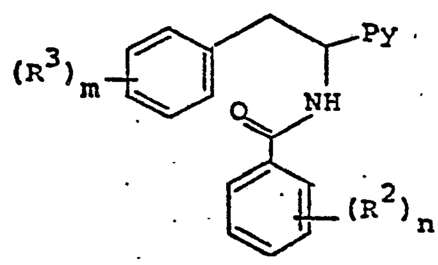

(II)

in which m, n, $R^2$ and $R^3$ have the meanings indicated for the formula I, and Py denotes the 3- or 4-pyridyl radical, with a dehydrating agent in a high-boiling solvent at a temperature between 100 and 220 °C to give isoquinoline derivative of the formula III

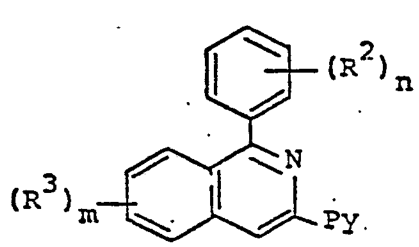

(III)

then reacting the compound thus obtained of the formula III with an alkylating agent Z—$R^4$, in which Z denotes iodine, bromine, chlorine or the mesyl or tosyl radical, and $R^4$ denotes benzyl or a straight-chain or branched, $C_1$-$C_6$-alkyl radical, or a straight-chain or branched alkenyl radical with up to 6 carbon atoms, and the quaternary pyridinium salt thus obtained is reduced with a complex metal hydride to give a compound of the formula I in which the radicals $R^1$ together represent a bond, and $R^4$ denotes benzyl or a straight-chain or branched, $C_1$-$C_6$-alkyl radical or a straight-chain or branched alkenyl radical with up to 6 carbon atoms, or

b) catalytically reducing a compound of the formula III to give a compound of the formula I in which m, n, $R^2$, $R^3$, A and B have the meanings mentioned for formula I, and the radicals $R^1$ and $R^4$ equal hydrogen, and optionally reacting a compound of the formula I thus obtained with an alkylating agent of the formula Z—$R^4$ to give a compound of the formula I in which m, n, $R^2$, $R^3$, A and B have the meanings indicated for formula I, and the radicals $R^1$ denote hydrogen, and $R^4$ denotes benzyl or a straight-chain or branched $C_1$-$C_6$-alkyl radical or a straight-chain or branched alkenyl radical with up to 6 carbon atoms.

3. Pharmaceutical composition containing a 1-phenylisoquinoline derivative of the formula I in claim 1.

4. 1-Phenylisoquinoline derivatives of the general formula I in claim 1 for use in treating depressions.

**Claim** (for the Contracting state AT)

A process for the preparation of 1-phenylisoquinoline derivatives of the formula I

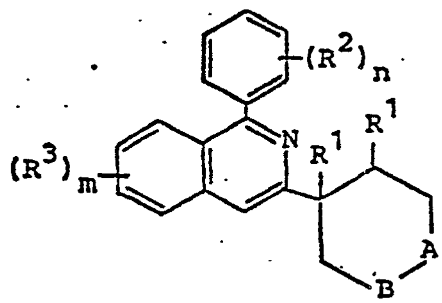

(I)

in which

m and n, independently of one another, denote one or two,

A and B denote a $CH_2$ or N—$R^4$ group, $R^4$ being hydrogen, benzyl or a straight-chain or branched $C_1$-$C_6$-alkyl radical or a straight-chain or branched alkenyl radical with up to 6 carbon atoms, and the radicals $R^1$ denote hydrogen or, together, a bond, and

$R^2$ denotes hydrogen, halogen, hydroxyl, nitro, amino, $C_1$-$C_6$-alkyl or $C_1$-$C_6$-alkoxy radicals, and

$R^3$ denotes hydrogen, halogen, hydroxyl, nitro, amino, $C_1$-$C_6$-alkyl or $C_1$-$C_6$-alkoxy radicals, or the benzyloxy, methylenedioxy or ethylenedioxy group, which comprises

a) reacting a compound of the formula II

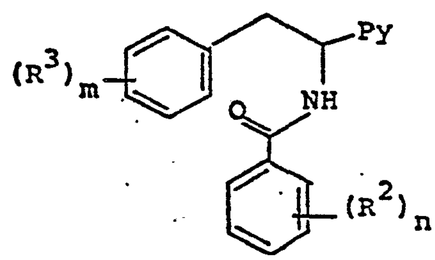

(II)

in which m, n, $R^2$ and $R^3$ have the meanings indicated for the formula I, and Py denotes the 3- or 4-pyridyl radical, with a dehydrating agent in a high-boiling solvent at a temperature between 100 and 220 °C to give an isoquinoline derivative of the formula III

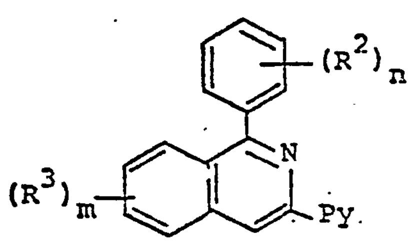

(III)

then reacting the compound thus obtained of the formula III with an alkylating agent Z—$R^4$, in which Z denotes iodine, bromine, chlorine or the mesyl or tosyl radical, and $R^4$ denotes benzyl or a straight-chain or branched $C_1$-$C_6$-alkyl radical or a straight-chain or branched alkenyl radical with up to 6 carbon atoms, and the quaternary pyridinium salt thus obtained is reduced with a complex metal hybride to give a compound of the formula I in which the radicals $R^1$ together represent a bond, and $R^4$ denotes benzyl or a straight-chain or branched $C_1$-$C_6$-alkyl radical or a straight-chain or branched alkenyl radical with up to 6 carbon atoms, or

b) catalytically reducing a compound of the formula III to give a compound of the formula I in which m, n, $R^2$, $R^3$, A and B have the meanings mentioned for formula I, and the radicals $R^1$ and $R^4$ equal hydrogen, and optionally reacting a compound of the formula I thus obtained with an alkylating agent of the formula Z—$R^4$ to give a compound of the formula I in which m, n, $R^2$, $R^3$, A and B have the meanings indicated for formula I, and the radicals $R^1$ denote hydrogen, and $R^4$ denotes benzyl or a straight-chain or branched $C_1$-$C_6$-alkyl radical or a straight-chain or branched alkenyl radical with up to 6 carbon atoms.

**Revendications** (pour les Etats contractants : BE, CH, DE, FR, GB, IT, LI, LU, NL, SE)

1. Dérivés de la 1-phénylisoquinoléine de formule générale I

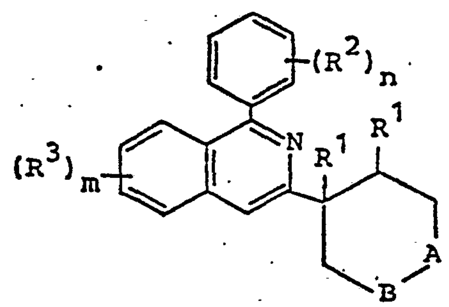

(I)

dans laquelle

m et n sont chacun, indépendamment l'un de l'autre, 1 ou 2 ;

A et B représentent chacun un groupe $CH_2$ ou N—$R^4$, $R^4$ étant un atome d'hydrogène, un radical benzyle, un reste alkyle en $C_{1-6}$ à chaîne droite ou ramifiée, ou un reste alcényle à chaîne droite ou ramifiée ayant jusqu'à 6 atomes de carbone ; les radicaux

$R^1$ représentent chacun un atome d'hydrogène ou ensemble une liaison, et

$R^2$ représente un atome d'hydrogène, un halogène, un groupe hydroxy, nitro, amino, un reste alkyle en $C_{1-6}$ ou alcoxy en $C_{1-6}$ ; et

$R^3$ représente un atome d'hydrogène, un halogène, un groupe hydroxy, nitro, amino, un reste alkyle en $C_{1-6}$ ou alcoxy en $C_{1-6}$, un groupe benzyloxy, méthylènedioxy ou éthylènedioxy.

2. Procédé pour la préparation d'un composé de formule générale 1 dans la revendication 1, caractérisé en ce que

a) on fait réagir un composé de formule générale II

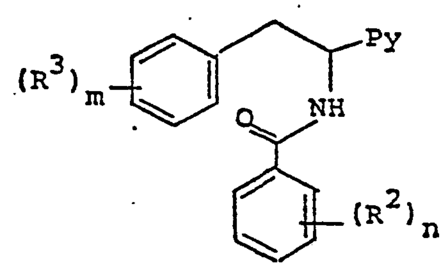

(II)

dans laquelle m, n, $R^2$ et $R^3$ ont les significations données pour la formule générale I et Py représente le reste 3- ou 4-pyridyle, avec un déshydratant dans un solvant à point d'ébullition élevé, à une température comprise entre 100 et 220 °C, pour aboutir à un dérivé de l'isoquinoléine, de formule générale III,

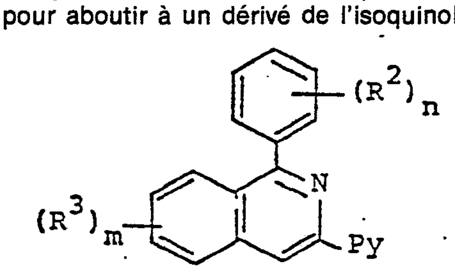

(III)

on fait réagir ensuite le composé de formule générale III ainsi obtenu avec un agent d'alkylation Z—$R^4$, dans lequel Z représente l'iode, le brome, le chlore, le reste mésyle ou tosyle, et $R^4$ représente le radical benzyle, un reste alkyle en $C_{1-6}$ à chaîne droite ou ramifiée, ou un reste alcényle à chaîne droite ou ramifiée contenant de 2 à 6 atomes de carbone, puis on réduit le sel de pyridinium quaternaire ainsi obtenu, avec un hydrure métallique complexe, pour aboutir à un composé de formule générale I dans lequel les restes $R^1$ représentent ensemble une liaison et $R^4$ représente le radical benzyle, un reste alkyle en $C_{1-6}$ à chaîne droite ou ramifiée, ou un reste alcényle en $C_{2-6}$ à chaîne droite ou ramifiée,
ou

b) on réduit par voie catalytique un composé de formule générale III pour obtenir un composé de formule I dans lequel m, n, $R^2$, $R^3$, A et B ont les significations données pour la formule I et les restes $R^1$ et $R^4$ sont chacun un atome d'hydrogène, et on fait éventuellement réagir un composé de formule générale I ainsi obtenu, avec un agent d'alkylation de formule générale Z—$R^4$ pour obtenir un composé de formule générale I dans lequel m, n, $R^2$, $R^3$, A et B ont les significations données pour la formule I, et le reste $R^1$ représente un atome d'hydrogène, et $R^4$ un radical benzyle, un reste alkyle en $C_{1-6}$ à chaîne droite ou ramifiée, ou un reste alcényle à chaîne droite ou ramifiée contenant jusqu'à 6 atomes de carbone.

3. Médicament caractérisé par une teneur en un dérivé de la 1-phénylisoquinoléine de formule générale I dans la revendication 1.

4. Dérivés de la 1-phénylisoquinoléine de formule générale I dans la revendication 1, pour l'utilisation dans le traitement de dépressions.

**Revendication** (pour l'Etat contractant AT)

Procédé pour la préparation de dérivés de la 1-phénylisoquinoléine de formule générale I

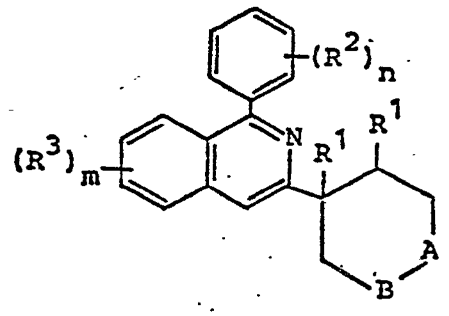

(I)

dans laquelle

m et n sont chacun, indépendamment l'un de l'autre, 1 ou 2 ;

A et B représentent chacun un groupe $CH_2$ ou N—$R^4$, $R^4$ étant un atome d'hydrogène, un radical benzyle, un reste alkyle en $C_{1-6}$ à chaîne droite ou ramifiée, ou un reste alcényle à chaîne droite ou ramifiée ayant jusqu'à 6 atomes de carbone ; les radicaux

$R^1$ représentent chacun un atome d'hydrogène ou ensemble une liaison, et

$R^2$ représente un atome d'hydrogène, un halogène, un groupe hydroxy, nitro, amino, un reste alkyle en $C_{1-6}$ ou alcoxy en $C_{1-6}$ ; et

$R^3$ représente un atome d'hydrogène, un halogène, un groupe hydroxy, nitro, amino, un reste alkyle en $C_{1-6}$ ou alcoxy en $C_{1-6}$, un groupe benzyloxy, méthylènedioxy ou éthylènedioxy, caractérisé en ce que

a) on fait réagir un composé de formule générale II

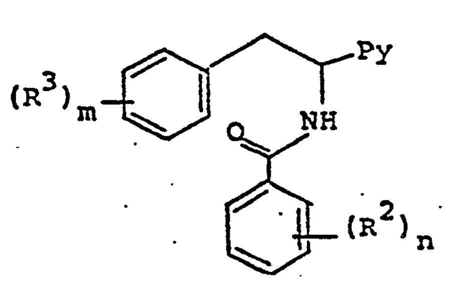

(II)

dans laquelle m, n, $R^2$ et $R^3$ ont les significations données pour la formule générale I et Py représente le reste 3- ou 4-pyridyle, avec un déshydratant dans un solvant à point d'ébullition élevé, à une température comprise entre 100 et 220 °C, pour aboutir à un dérivé de l'isoquinoléine, de formule générale III,

(Siehe Figur Seite 17 f.)

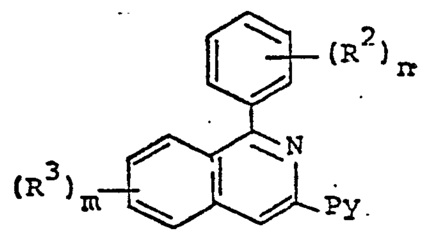

(III)

on fait réagir ensuite le composé de formule générale III ainsi obtenu avec un agent d'alkylation Z—$R^4$, dans lequel Z représente l'iode, le brome, le chlore, le reste mésyle ou tosyle, et $R^4$ représente le radical benzyle, un reste alkyle en $C_{1-6}$ à chaîne droite ou ramifiée, ou un reste alcényle en $C_{2-6}$ à chaîne droite ou ramifiée, puis on réduit le sel de pyridinium quaternaire ainsi obtenu, avec un hydrure métallique complexe, pour aboutir à un composé de formule générale I dans lequel les restes $R^1$ représentent ensemble une liaison et $R^4$ représente le radical benzyle, un reste alkyle en $C_{1-6}$ à chaîne droite ou ramifiée, ou un reste alcényle en $C_{2-6}$ à chaîne droite ou ramifiée,
ou

b) on réduit par voie catalytique un composé de formule générale III pour obtenir un composé de formule I dans lequel m, n, $R^2$, $R^3$, A et B ont les significations données pour la formule I et les restes $R^1$ et $R^4$ sont chacun un atome d'hydrogène, et on fait éventuellement réagir un composé de formule générale I ainsi obtenu, avec un agent d'alkylation de formule générale Z—$R^4$ pour obtenir un composé de formule générale I dans lequel m, n, $R^2$, $R^3$, A et B ont les significations données pour la formule I, et le reste $R^1$ représente un atome d'hydrogène, et $R^4$ un radical benzyle, un reste alkyle en $C_{1-6}$ à chaîne droite ou ramifiée, ou un reste alcényle à chaîne droite ou ramifiée contenant jusqu'à 6 atomes de carbone.